# EUROPEAN PATENT SPECIFICATION

(11) **EP 4 051 156 B1**
(45) Date of publication and mention of the grant of the patent: **03.06.2026**
(21) Application number: 20882195.9
(22) Date of filing: 29.11.2020
(51) Int. Cl.: A61B 34/10, G06T 7/10

(54) **METHOD FOR PLANNING AN ORTHOPEDIC PROCEDURE**
VERFAHREN ZUR PLANUNG EINES ORTHOPÄDISCHEN EINGRIFFS
PROCÉDÉ POUR LA PLANIFICATION D'UNE INTERVENTION ORTHOPÉDIQUE

(30) Priority: 29.10.2019 SE 1951237
(43) Date of publication of application: 07.09.2022
(73) Proprietor: Ortoma AB, 412 85 Gothenburg (SE)
(72) Inventor: PETTERSSON, Andreas, 435 40 Mölnlycke (SE); MOLYNEUX, Peter, 443 38 Lerum (SE)
(74) Representative: Ström & Gulliksson AB
(86) International application number: PCT/SE2020/051143
(87) International publication number: WO 2021/086260

(56) References cited:
- EP-A1- 2 870 941
- EP-A2- 3 273 375
- WO-A1-00/56215
- GB-A- 2 393 625
- US-A1- 2008 262 812
- US-A1- 2008 269 906
- US-A1- 2010 153 081
- US-A1- 2012 008 848
- US-A1- 2014 093 153
- US-A1- 2015 324 114
- US-A1- 2018 008 350
- US-A1- 2018 085 165
- US-A1- 2019 090 952
- US-A1- 2019 239 926
- US-A1- 2019 239 926
- US-A1- 2019 298 452
- US-B1- 8 644 568

## Description

### Field of the Invention

This invention pertains in general to the field of orthopedics, such as hip, knee, spine, shoulder, trauma or extremity orthopedic procedures. More particularly, the invention relates to a computer implemented method and apparatus for planning an orthopedic procedure. The method comprises retrieving medical imaging data that comprises a portion of a first bone and a portion of a second bone. In the retrieved medical imaging data, the portion of the first bone and the portion of the second bone are unsegmented, i.a. the portion of the first bone and the portion of the second bone are not labeled in the medical imaging data such that the may be identified separately. The portion of the first bone and the portion of the second bone are segmented such that the portion of the first bone is moveable relative the portion of the second bone portion. A plurality of landmarks including at least a first landmark at the portion of the first bone and at least a second landmark at the portion of the second bone may be identified. This may be done before or after the segmentation, or even without performing the segmentation. At least one of a first implant component and a second implant component may be selected from among a plurality of implant components in a database based on information obtained from the first landmark and the second landmark. The first implant component and/or the second implant component may be fitted in a space at least partially defined by the first landmark and the second landmark.

### Background of the Invention

Orthopedic procedures, such as such as hip, knee, spine, shoulder, trauma or extremity orthopedic procedures are may be digitally planned using digital medical imaging data. For example, the type, size, and position of implant components relative to medical imaging data can be planned pre-operatively, i.e. before the patient enters the operating room, or intra-operatively, i.e. when the patient has entered the operating room. Planning the type, size, and position of implant components relative to medical imaging data is also referred to as templating. Traditionally, this was performed using 2D imaging data, but more modern approaches uses 3D imaging data for planning of orthopedic procedures.

The medical imaging data may come from various sources, such as X-ray, fluoroscopy, CT (Computer Tomography), CBCT (Cone Beam Computer Tomography), Ultrasound, and MRI (Magnetic Resonance Imaging).

A challenge with planning of orthopedic implant procedures is that the relative position of various bones of the patient when scanning the patient to generate the medical imaging data may not correspond to the relative positions the bones will have during the operation or the relative positions the bones will have after the operation when the implants have been placed. For example, a hip surgery, such as THA (Total Hip Arthoplasty), involving restoration of the patient's biomechanics may include lengthening/shortening of the leg, adjusting various offsets, etc. For example, it may be desired to move the femur relative to the pelvis of the patient in order to obtain better biomechanics. This can be done by selecting implant components with appropriate types and sizes to achieve the desired biomechanics. However, selecting the correct implant components to achieve the desired outcome of the procedure is difficult since the femur and the pelvis have a fixed positional relationship in the medical imaging data when unsegmented and does not represent the desired relative position to obtain the desired biomechanics. This is even more difficult when multiple components are selected, that will not have the same interrelationship on screen when planning is performed as after surgery in view of the unsegmented character of the imaging data. Similar challenges apply e.g. for a TKA (Total Knee Arthroplasty) or PKA (Partial Knee Arthroplasty) procedures, shoulder procedures, etc., where relative bone positions are modified as a part of the orthopedic procedure.

In some types of procedures, the position of the patient in the medical imaging scanner, where the medical imaging data is captured, may not correspond to the position of the patient on the operating table. This is e.g. the case for spine surgery, where the medical imaging data is captured in a CT or MRI scanner pre-operatively with the patient in supine position, whereas during surgery the patient is in lateral or prone position. The vertebras of the spine inevitably have different interrelationships in the various positions. For example, for pedicle screw fixation the relative position of the vertebras at which they should be fixed is obtained when the patient is lying on the operating table. A 2D or 3D C-arm may be used during surgery, e.g. to identify the entry level of the spine, and/or to match the pre-operative image data to the intra-operative image data to use pre-operatively planning data for navigation or robotic surgery. However, since the vertebras have different interrelationships in the two sets of data, such matching may be challenging or impossible. This may have the consequence that it is not possible to plan pre-operatively, or the sub-optimal implants or implant positions are planned. US20190090952 relates to a computerized method to plan a total knee arthroplasty procedure, in which medical image data is segmented to generate 3-D models of the bones and anatomical landmarks are identified on the segmented models of the bone. The system and method automatically aligns the implant components and the bones according to a desired clinical alignment goal with minimum user input. The system and method further allows the user to adjust the position and orientation of the bones that in the context of a knee arthroplasty are the femur, tibia, or implant in a clinical direction regardless of a pre-adjusted position and orientation of the femur, tibia, or implant.

In summary, the interrelationships of bones in the medical imaging data may not be optimal for performing the planning and/or surgery and may introduce inaccuracies in the planning or surgery or even lead to selecting sub-optimal implants. In worst case, implants are placed in sub-optimal positions, which may even be unsafe for the patient.

Hence, an improved method for planning an orthopedic procedure would be advantageous and in particular improved precision, increased flexibility, cost-effectiveness, and/or patient safety would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a method, an apparatus, and computer program product for planning an orthopedic procedure. Embodiments of the invention are defined by the following detailed description and the appended patent claims.

Some embodiments comprise a computer implemented method for planning an orthopedic procedure. The method comprises retrieving medical imaging data comprising a portion of a first bone and a portion of a second bone, wherein the portion of the first bone and the portion of the second bone are unsegmented. A plurality of landmarks including at least a first landmark at the portion of the first bone and at least a second landmark at the portion of the second bone are identified.

The portion of the first bone and the portion of the second bone may be segmented, such that the portion of the first bone is moveable relative the portion of the second bone.

A first implant component and a second implant component may be selected from among a plurality of implant components in a database based on information obtained from the first landmark and/or the second landmark.

At least one of the first implant component and the second implant component may be fitted in a space at least partially defined by the first landmark and the second landmark.

In some embodiments, the method comprises moving the portion of the first bone relative to the portion of the second bone based on at least one landmark of the plurality of landmarks after segmenting the portion of the first bone and the portion of the second bone. Alternatively or additionally, the method comprises comprising moving the portion of the first bone relative to the portion of the second bone based on a contour of the segmented portion of the first bone and/or a contour of the segmented portion of the second bone.

In some embodiments, the portion of the first bone is moved relative to the portion of the second bone to obtain a desired position of the first landmark relative to a position of the second landmark before selecting a first implant component and/or a second implant component.

The information obtained from the first landmark and/or the second landmark may be obtained after segmenting the portion of the first bone and the portion of the second bone and after the portion of the first bone is moved relative to the portion of the second bone.

The first landmark and the second landmark may be landmarks of the portion of the first bone or the portion of the second bone. Alternatively, the first landmark is a landmark of the portion of the first bone and the second landmark is a landmark of the portion of the second bone.

Embodiments of the method may comprise identifying a third landmark of at least one of the portion of the first bone and the portion of the second bone, and obtaining the information based of the first landmark, the second landmark, and the third landmark.

Obtaining the information from the first landmark and the second landmark may comprise obtaining at least one dimension, which includes at least one of diameter, length, width, and angle.

Embodiments may comprise storing each implant component in the database together with implant information, which optionally may include at least one of diameter, length, width, and angle.

Selecting at least one of the first implant component and the second implant component may comprise selecting an implant component having implant information with a best fit to the information obtained from the first landmark and the second landmark.

Embodiments comprise a computer readable storage medium, which has program instructions stored therein, wherein the program instructions, when executed by a processor, perform the method of the embodiments described herein.

Embodiments comprise an apparatus for planning an orthopedic procedure. The apparatus is configured to access a memory to retrieve medical imaging data comprising a portion of a first bone and a portion of a second bone, wherein the portion of the first bone and the portion of the second bone are unsegmented, using a processing unit to identify a plurality of landmarks including at least a first landmark at the portion of the first bone and at least a second landmark at the portion of the second bone, using the processing unit to segment the portion of the first bone and the portion of the second bone, such that the the portion of the first bone is moveable relative the portion of the second bone portion, using the processing unit to select at least one of a first implant component and a second implant component from among a plurality of implant components in a database based on information obtained from the first landmark and the second landmark, and using the processing unit to fit at least one of the first implant component and the second implant component in a space at least partially defined by the first landmark and the second landmark.

The processing unit may be configured to move the portion of the first bone relative to the portion of the second bone based on at least one landmark of the plurality of landmarks after segmenting the portion of the first bone and the portion of the second bone, and/or based on a contour of the segmented portion of the first bone and/or a contour of the portion of the second bone.

The processing unit may be configured to move the portion of the first bone relative to the portion of the second bone to obtain a desired position of the first landmark relative to a position of the second landmark before selecting a first implant component and/or a second implant component.

The processing unit may be configured to obtain the information from the first landmark and the second landmark after segmenting the portion of the first bone and the portion of the second bone and after the portion of the first bone is moved relative to the portion of the second bone.

Further embodiments of the invention are defined in the dependent claims.

Some embodiments of the invention provide for efficient and accurate planning of an orthopedic procedure. Since portions of bones are segmented, it makes it possible to move bone portions relative to each other, such that the actual position of the bone portions may be moved to a desired relative to positions to simulate a desired outcome of a treatment. After this has been done, implant components may be selected. This optimizes selection of implant components to fit a desired outcome of a surgery, even before the surgery commences. Furthermore, segmented bone portions may be rendered in different colors, which may also facilitate planning the procedure. Also, landmarks on the bone portions may be identified. The landmarks may be used to select implant components. Furthermore, the landmarks may be used to move portions of bone relative to each other in order to plan the treatment. For example, the landmarks can be used to plan for biomechanical symmetry or biomechanical restoration by moving bone portions. This is possible due to combination with segmentation of the bone portions. Hence, identification of landmarks also contributes to efficient and accurate planning of an orthopedic procedure.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a flow chart of embodiments of a method for planning an orthopedic procedure;
Fig. 2 is a block diagram of an apparatus for planning an orthopedic procedure;
Figs. 3-6 are schematic views of embodiments of user interfaces for user input and manipulation wherein example landmarks of medical imaging data are identified; and
Figs. 7-8 are schematic views of embodiments of user interfaces illustrating portions of bones that have been moved relative to each other and where implant components have been fitted into a space defined by landmarks.

### Description of Embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on embodiments of the present invention applicable for planning an orthopedic procedure exemplified by a planning of a THA (Total Hip Arthroplasty). However, it will be appreciated that the invention is not limited to this application but may be applied to many other procedures, such as including knee, spine, shoulder, extremities, and trauma orthopedic procedures, particularly wherein orthopedic implants are used.

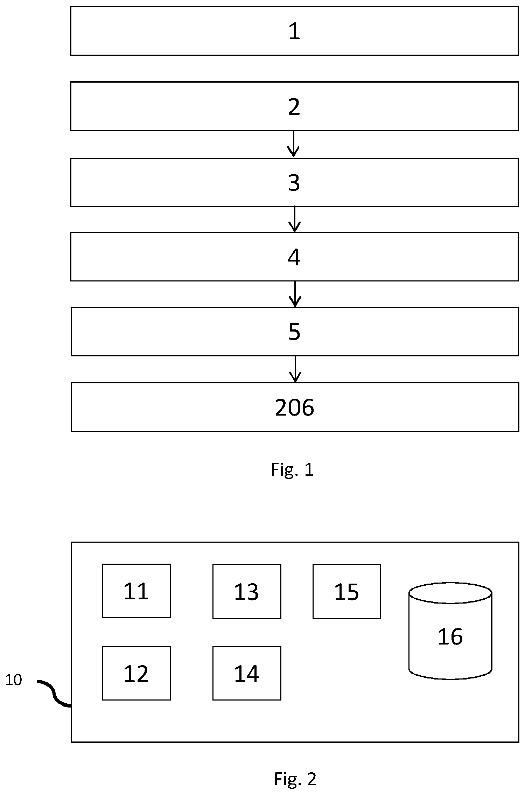

Figs. 1-2 illustrate embodiments of a computer implemented method and an apparatus 10 for planning an orthopedic procedure.

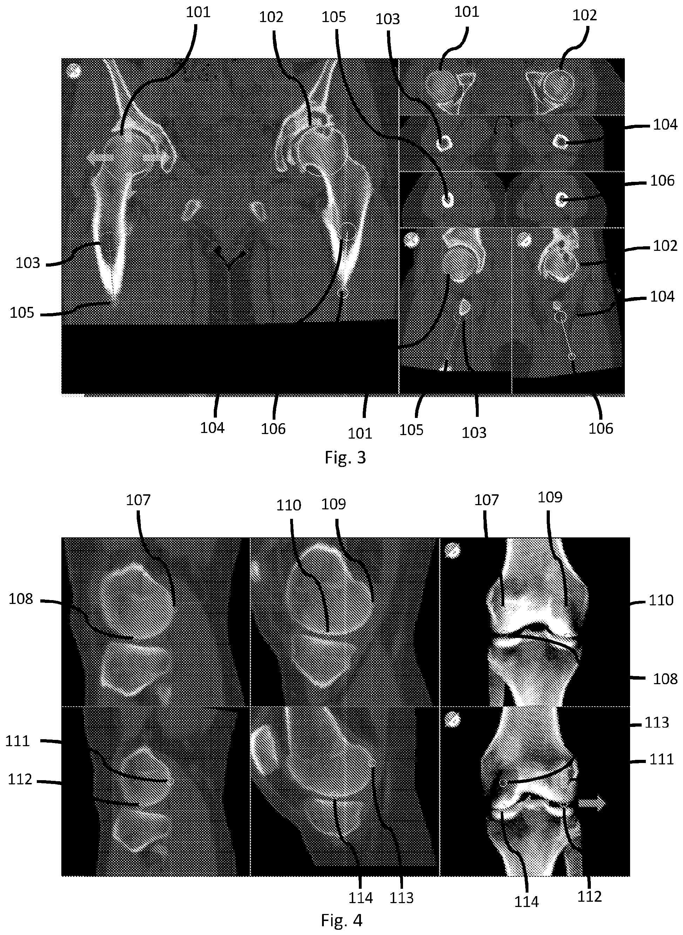

According to the method illustrated in Fig. 1, medical imaging data is retrieved 1. The medical imaging data may comprise a portion of a first bone and a portion of a second bone. Further bone portions may also be included. The portion of the first bone and the portion of the second bone may be unsegmented when retrieved, such as from a storage media. Then, at least one landmark 100-121 (illustrated in Figs. 3-6), preferably a plurality of landmarks, is identified 2. The landmarks 100-121 may include at least a first landmark at the portion of the first bone and at least a second landmark at the portion of the second bone. Once the landmarks have been identified, the imaging data including the landmarks 100-121 may be stored in memory or presented on the screen.

Identifying 2 landmarks 100-121 may be performed manually by a user providing input to a computer by selecting portions or areas on the medical imaging data when presented on a screen. Alternatively or additionally, the landmarks 100-121 may be identified by an analytical model, which may be computer implemented. Such analytical model may be set up using a machine learning model that has been trained via an iterative learning process. For example, the analytical model may be set up using a neural network that can cluster and recognize patterns in the imaging data. In a learning process, the landmarks 100-121 are defined, and then a user defines, via user interaction, where the landmarks are located within the imaging data. Gradually, the analytical model may continuously learn and improve in order to more precisely identify the landmarks 100-121 in the medical imaging data. Once fully trained, the analytical model may recognize the landmarks 100-121 at great precision without human interaction. Yet, human confirmation or enhancement of accuracy may be desired as will be further discussed below.

The imaging data may comprise at least one of X-ray, fluoroscopy, CT (Computer Tomography), CBCT (Cone Beam Computer Tomography), Ultrasound, and MRI (Magnetic Resonance Imaging) and be generated by a medical imaging device. When generated, the medical imaging data is unclassified or unlabeled, such that one bone of the patient cannot be distinguished from another bone of the patient. Therefore, one bone of the patient cannot be moved relative to the other without further processing. The process of distinguishing one bone from the other is generally referred to as segmentation.

The method may comprise segmenting 3 the medical imaging data. Segmenting 3 the imaging data may be performed before or after identifying 2 the landmarks 100-121. In some embodiments, segmentation may even be performed independently from the identification of landmarks 100-121. According to embodiments, segmenting 3 the medical imaging data may comprise dividing that medical imaging data into at least two data sets. This may be used for moving a portion of the first bone relative to relative said portion of the second bone portion, as will be further illustrated in embodiments described below. Segmentation may be performed manually by a user. Alternatively or additionally an analytical model, e.g. using machine learning similar to the model for identifying landmarks may be implemented. A combination approach may also be used, where the analytical model first prepares a suggested segmentation, which may be corrected by the user by tagging certain medical imaging data as belonging to a particular portion of bone. At the most detailed level, each voxel in a set of medical imaging data can be tagged as belonging to a particular bone.

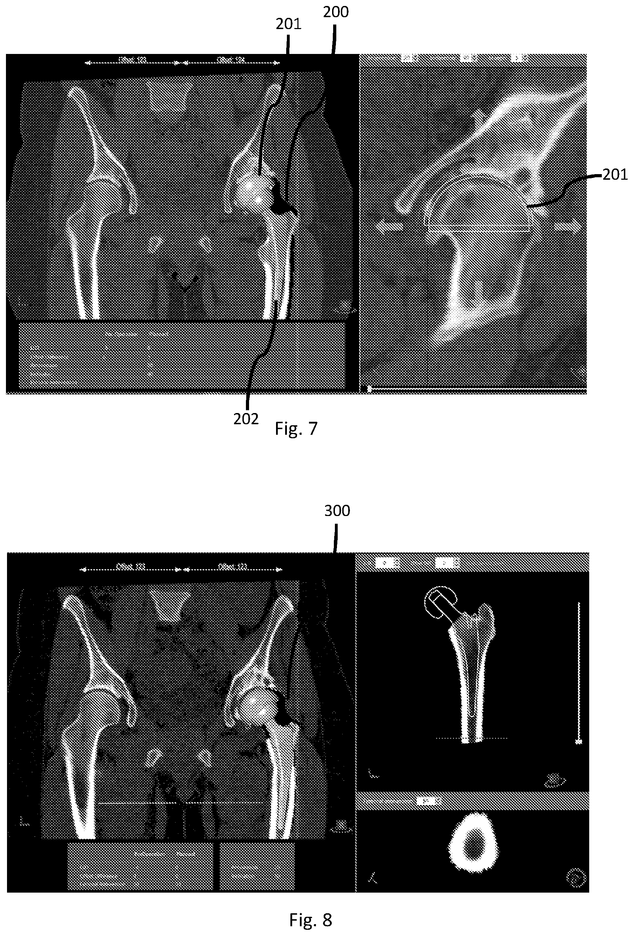

According to embodiments, the method may also comprise selecting 4 at least one implant component 201, 202 (Fig. 7-8) based on information obtained from at least one landmark, such as the first landmark and the second landmark. Some embodiments comprise selecting 4 a plurality of implant components, such as at least a first implant component and a second implant component from among a plurality of implant components. The implant component(s) 201, 202 may be selected 1 from a plurality of implant components in a database based on the information obtained from the first landmark and the second landmark. Selecting 4 the implant component(s) 201, 202 from the database based on information obtained from at least one landmark 100-121 may be performed with or without the segmentation 3 described above.

After an appropriate implant component or implant components 201, 202 have been selected 4, the implant component(s), such as the first implant component 201 and the second implant component 202, may be fitted 5 in a space at least partially defined by the landmark(s), as will be further exemplified with regard to embodiments described below. Hence, at least one landmark that was used for selecting 4 may also be used for fitting 5 the implant component 201, 202 in the space defined by the landmark(s).

Fig. 2 illustrates embodiments of an apparatus 10 for planning an orthopedic procedure. The apparatus 10 may comprise a computer system, into which the medical imaging data may be retrieved 1 and the surgical procedure planned. In case the imaging data is generated during surgery, the apparatus 10 may also be used intra-operatively. The result of the planning may be made available for other medical devices and systems, such as a navigation system, a robotic system, or for generating patient specific implants. For example, for navigation or surgical systems, the positional information for the implant component(s) relative to the medical imaging data may be used to guide the surgeon visibly via a display, or to control a robotic arm. The apparatus 10 may comprise a CPU/processor or data processing unit 11, one or several memories 12, a communication unit 13, an input device 14, such as a mouse and/or a keyboard for user interaction, and an output unit 15, such as a display in which the medical imaging data, landmarks, etc. may be rendered. Imaging data, models of implant components, and information concerning the imaging data and the implant components may be stored in a storage media, such as the database 16 and/or the memories 12. The database 16 may be a local database or a network resource that multiple apparatuses 10 according to the invention can access. The medical imaging data can be uploaded into the storage media from the medical scanner, in which the data is generated. The processing unit may access the memory to retrieve data based on user input as is necessary throughout a planning session.

Computer software in which the present invention is implemented when run by the CPU 11 may comprise a CAD system, wherein the medical imaging data can be rendered, such as a 3D model of the surgical object or the 3D volume of scan data, and/or multiple 2D views of scan data, such as MRI or CT data, generated from a 3D volume of medical imaging data. A 2D/3D model of the implant component(s) and 2D views of medical imaging data may also be rendered at the same time and be partially overlaid in order to increase the information.

Figs. 3-6 illustrate rendering of a volume of 3D medical imaging data, such as CT, CBCT, MRI, or ultra-sound data in multiple views. Landmarks 100-121 are indicated as illustrative examples. The landmarks may be identified 2 as discussed above, and presented on a screen or a display in multiple 2D views. The 2D views may e.g. be cross-sectional views or projections of a 3D volume. By rendering the data in multiple views generated from orthogonal views of a volume of medical imaging data facilitates user interaction. In each of the views, the user can adjust the position of the landmarks 100-121 in only one or two dimensions. Since the same landmark 100-121 is rendered in several of the views, at a different angle in each view, user inaction in at least two views by repositioning the landmark 100-121 may redefine the position in three dimensions relative to the medical imaging data. A more detailed description of rendering of the medical imaging data in multiple 2D views is explained in WO2014062125. Hence, identifying the landmarks may comprise user input that identifies the exact location of the landmark relative to the medical imaging data. Hence, user input may fine tune the identification and make it more accurate. This will make the process for identifying the landmarks 100-121 more reliable. As a safety measure, the user may need to verify the portion of each landmark before the plan can ultimately be approved.

As discussed above, embodiments of the present invention may comprise identifying 2 a plurality of landmarks 100-121, such as anatomical landmarks of a bone portion of the medical imaging data. Figs. 3-6 illustrate various anatomical landmarks in multiple 2D views. The landmarks are illustrative embodiments. In other embodiments, some of the landmarks illustrated in Figs. 3-6 are identified 2. In other embodiments, additional or other landmarks of the human body may be identified depending on the type of orthopedic procedure and the type of implant component that is to be planned.

Fig. 3 illustrates identification of a plurality of landmarks 100-106 of portions of bone including at least a portion of the right and left femur, the landmarks comprising:
- right femur head 100 (left view, top left landmark; right top view, left landmark; 2^{nd} right bottom view, top landmark);
- left femur head 102 (left view, top right landmark; right top view, right landmark; 1^{st} right bottom view, top landmark);
- right proximal femur shaft 103 (left view, center left landmark; 2^{nd} right top view, left landmark; 2^{nd} right bottom view, center landmark); right proximal femur shaft 103 may be identified in the femoral canal at the level of the lesser trochanter of the femur;
- left proximal femur shaft 104 (left view, center right landmark; 2^{nd} right top view, right landmark; 1^{st} right bottom view, center landmark); left proximal femur shaft 104 may be identified in the femoral canal at the level of the lesser trochanter of the femur;
- right distal femur shaft 105 (left view, bottom left landmark; 3^{rd} right top view, left landmark; 2^{nd} right bottom view, bottom landmark); right distal femur shaft 105 may be identified a predefined distance from the right proximal femur shaft 103;
- left distal femur shaft 106 (left view, bottom right landmark; 3^{rd} right top view, right landmark; 1^{st} right bottom view, bottom landmark); left distal femur shaft106 may be identified a predefined distance from the left proximal femur shaft 104;

Fig. 4 illustrates identification of a plurality of landmarks of portions of bone including at least a portion of the right and left femur and the right and left tibia, the landmarks comprising:
- right lateral posterior condyle 107 (top left view, top landmark; top right view, top left landmark);
- 108 right lateral distal condyle (top left view, bottom landmark; top right view, bottom left landmark);
- 109 right medial posterior condyle (top center view, top landmark; top right view, top right landmark);
- 110 right medial distal condyle (top center view, bottom landmark; top right view, bottom right landmark);
- 111 left lateral posterior condyle (bottom left view, top landmark; bottom right view, top left landmark);
- 112 left lateral distal condyle (bottom left view, bottom landmark; bottom right view, bottom right landmark);
- 113 left medial posterior condyle (bottom center view, top landmark; bottom right view, top left landmark);
- 114 left medial distal condyle (bottom center view, bottom landmark; bottom right view, bottom left landmark);

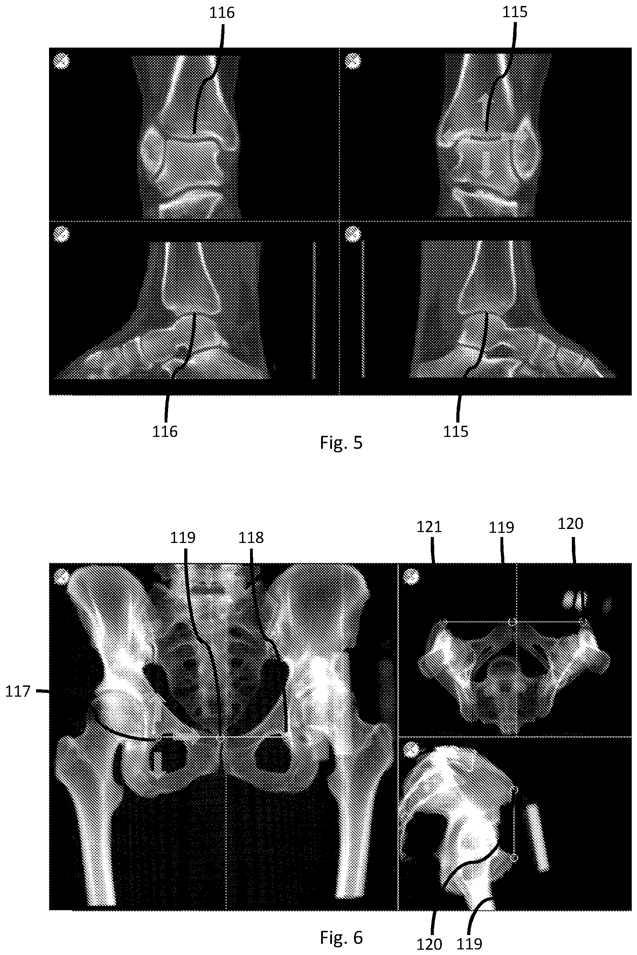

Fig. 5 illustrates identification of a plurality of landmarks of portions of bone including at least a portion of the right and left tibia and the right and left tibia, the landmarks comprising:
- 115 left tibia (right top and bottom views);
- 116 right tibia (left top and bottom views);

Fig. 6 illustrates identification of a plurality of landmarks of portions of bone including at least a portion of the pelvis, the landmarks comprising:
- right acetabular notch 117 (left view, left landmark);
- left acetabular notch 118 (left view, right landmark);
- center of pubic tubercle 119 (left view, center landmark; right top view, center landmark);
- right SIAS (spina iliaca anterior superior) 120 (right top view, right landmark; right bottom view, top landmark)
- left SIAS 121 (right top view, left landmark)

The landmarks 100-121 may be indicated in the medical imaging data in the view on a screen using an indicator, which may have a pre-defined shape. The shape may e.g. be a circle, a cross, a line, etc. The indicator may have a fixed size, such as a cross or a point, e.g. in order to indicate a landmark defined by a specific point on the portion of bone. Such a point may e.g. be a center of rotation or a specific point at a surface of the portion of bone. Landmarks 107-121 are such indicators having a fixed size. When the user click on one of the indicators in the shape of a circle, a cross appears in the center of the circle in order to facilitate positioning the indicator with great accuracy. Hence, the indicators may have multiple shapes. Alternatively, the size of the indicator may be adjustable, such as exemplified by the indicators identifying landmarks 101-106. For example, the indicators identifying the femur heads 101-102 may comprise a circle that is adjustable in diameter. The contour of the circle can be used to identify the contour of the femur head. At the same time, a cross at the center of the circle can identify the center of rotation of the femur head. Hence, one indicator may identify multiple landmarks of a single portion of bone. Other examples of indicators that can have adjustable sizes are the indicators for the femoral shafts 103-106. These landmarks may comprise a dimension as well as a position. In the illustrated examples, the proximal femur shaft is a landmark at the lesser trochanter and at which a diameter of the femur shaft can be indicated. The center of the indicator identifies the position within the femur shaft, whereas the diameter of the indicator identifies the diameter of the femur shaft at the particular positon. Similarly, the distal femur shaft 105, 106 can be indicated, wherein the position is defined at a predefined position from the proximal femur shaft landmark 103, 104. The dimeter of the circles can be set based on a particular value of the medical imaging data. Such a value of the medical imaging data may comprise a grey value, e.g. identifying cortical bone or the border between harder and softer bone. Hence, a landmark may be identified based on a particular shape condition of the portion of the bone. Additionally or alternatively, the landmark may be identified based on a condition, status or quality of the bone, such as relatively softer and relatively denser bone.

Adjusting the positon of the indicator for the landmark may be performed by user interaction/input. The analytical model may be used to propose a position of the landmark 100-121. A user may provide input to adjust the positon of an indicator of the landmark, e.g. by using an input device such as a mouse or a keyboard to move the indicator relative to the portion of bone on the screen. This is e.g. illustrated in Fig. 3 at landmark 101, in Fig. 4 at landmark 112, in Fig. 115 at landmark 115, and in Fig. 6 at landmark 117. Furthermore, the user may confirm that the landmarks 100-121 are ultimately correctly identified as a safety precaution and verification that all landmarks have been correctly identified by an analytical model.

As discussed above, bone portions may be segmented and sub-volumes generated. Each sub-volume may comprise one or several portions of bone. After segmentation, the bone portions may be stored as separate entities or separate sub-volumes. Sub-volumes may be manipulated separately. For example, on sub-volume may be moved individually relative to another sub-volume. Furthermore, one sub-volume may be rendered in a different color compared to another sub-volume. This makes it easier to distinguish between bone portions and verify the accuracy of the segmentation. On situation where it may be difficult to verify accuracy is for total hip arthroplasty due to arthritis. In such situations it is common that the kaput (femur head) lies directly against the acetabulum. This makes it difficult for the user to determine if the segmentation has been successful. Rendering the bone portions (the femur and the pelvis in this example), after segmentation, in different colors makes this determination easier.

Figs. 7-8 illustrates embodiments that comprises moving a portion of the first bone, exemplified by the pelvis, relative to a portion of the second bone, exemplified by the femur, based on at least one landmark 101-121 of the plurality of landmarks. These embodiments may be combined with the embodiments described above. Moving of the bone portions may be done after said segmenting the portion of the first bone and the portion of the second bone. Furthermore, multiple portions of bone may be moved as a unit relative to one or other portions of bone. In the example of Figs. 7-8, the femur and tibia have been moved relative to the pelvis. In Figs. 7-8, the femur and tibia have been moved such that the length of the left leg of the patient (for which the implant planning is performed in the example) has a length that is equal to the length of the left leg of the patient. This is also indicated in the table at the bottom of the left window of Fig. 7. Before moving the portion of first bone relative the portion of second bone, the leg length discrepancy (LLD) was -5mm (Fig. 7) and -4mm (Fig. 8), and after moving the LLD is 0mm. The length of the legs may be determined using the landmarks 101-121. The length of the legs may be calculated using the acetabular notches 117, 118, one or several landmarks of the knees 107-117 (which may be used to determine a center of the knees), and the landmarks at the ankles 115, 116. In the present case, there is a difference in length of the legs of the patient, which means that the leg subject for implant placement should be lengthened. For this purpose, the landmark at the ankles 115, 116 and/or the knees may be used. For example, the femur can be moved such that the center of the left knee is at the same transverse plane as the right knee. As mentioned, the centers of the knees may be determined by the landmarks 107-117. Additionally or alternatively, multiple portions of bone, such as the right femur and the right tibia, may be moved as a unit such that the landmark at the left ankle 115 is moved to the transverse plane at the landmark of the right ankle 116. The result of moving of the femur relative to the pelvis, i.e. the portion of the first bone relative to the portion of the second bone, based on the landmarks can be seen as a black silhouette 200, 300 in Figs. 7-8. The silhouette indicates where the femur used be before the femur was moved. Similarly, the bone portion(s) may be moved in the coronal plane and/or the sagittal plane of the patient.

Moving one or several portions of bone relative one or several other portions of bone may be useful for planning restoration of biomechanics, i.e. the desired outcome of the procedure, before implant components 201, 202 are selected and the positon of the components planned. Hence, the desired positions of the anatomy after surgery can be planned before the surgery actually commences. This follows more closely what happens in surgery, where bone portions are first positioned at a desired location, and then implant components 201, 202 are inserted to match that desired location. In Figs. 7-8, the implant components are exemplified by a cup/shell 201 and femur component 202 for a total hip arthroplasty.

Alternatively or additionally, rather than moving a portion of the first bone relative to a portion of the second bone (or multiple portions) based on the landmarks, the bone portions may be moved using a contour of the segmented portion of the first bone and/or a contour of the segmented portion of the second bone. For example, this may be useful for 3D-2D matching procedures, wherein a first set of medical imaging data of the patient is generated with a first medical imaging device, such as a CT or MR scanner, and one or several sets of medical imaging data of the patient is/are generated with a second medical imaging device, such as a fluoroscopy or x-ray scanner. The first set of medical imaging data, preferably in 3D, can be generated before surgery, and the second set(s) of medical imaging data, in 3D or 2D, can be generated during surgery. In some situations, the position of the patient in a medical imaging device for generating 3D medical imaging data before surgery is not the same as the position of the patient on the operating table. One such example is for spine surgery. 3D medical imaging data can be generated before surgery in a 3D medical imaging device, such as a CT or MR scanner, with the patient lying in supine position. However, in surgery, the patient may be lying in prone or lateral position. The positions of the vertebras relative to each other when the patient is lying in the supine position are not the same as when the patient is lying in prone or lateral position. Hence, the position of the bone portions in the 3D medical imaging data generated before surgery do not fully match the positions of the bone portions in the 2D/3D medical imaging data generated during surgery.

According to the present invention, it is still possible to plan the surgery before surgery. Alternatively or additionally, the surgery can be planned intra-operatively. If planed before surgery, the position of the implant component(s) is/are planned relative the 3D medical imaging data generated before surgery. The position of the bone portions in the 3D medical imaging data can be matched to positon of the bone portions in the 2D/3D medical imaging data generated intra-operatively. Hence, the bone portions in 3D medical imaging data generated before surgery can be moved. This may be based on the contour of the bone portions, which appears in the 3D medical imaging data generated pre-operatively as well as the 2D/3D medical imaging data generated intra-operatively. If necessary, the positions of the implant component(s) 201, 202, planned relative to the 3D medical imaging data generated pre-operatively can follow the movement and/or can be adjusted if necessary. For example, for a spine surgery pedicle screws can be planed relative to multiple portions of bone (vertebras). If a screw is only placed in a single vertebra, the screw can follow the movement. However, rods to be placed between the pedicle screws for fixating the vertebras relative to each other can be planned intra-operatively. Alternatively or additionally, the rods can be pre-operatively, and then be adjusted intra-operatively based on any movement of the bone portions.

Other embodiments when bone portions are moved are e.g. for knee surgery, wherein the tibia is moved relative to the femur before appropriate implant components and their relative positions in the tibia and/or femur are determined. Landmarks for knee implant surgery correspond to a large extent to landmarks for a hip implant surgery. Additional landmarks may e.g. comprise the canal of the tibia canal. For lumbar spine surgery, the entry point of the pedicle screw may be defined as the confluence of any of the four lines: pars interarticularis, the mamillary process, the lateral border of the superior articular facet, and/or the mid transverse process. Landmarks can be identified to define these lines. For thoracic spine surgery, the entry point of the pedicle screw for the distal thoracic segments may be defined after determining the intersection of the mid portion of the facet joint and the superior edge of the transverse process. The specific entry point can be just lateral and caudal to this intersection. The entry point tends to be more cephalad at more proximal thoracic levels. Landmarks may include the lateral border of the superior facet, the lateral border of the inferior facet, and/or the ridge of the pars interarticularis and the transverse process. These and other landmarks landmarks can be used to select an optimal implant component, such as pedicle screw with optimal length diameter, thread type, and/or thread pitch.

Hence, according to embodiments the portion of the first bone can be moved relative to the portion of the second bone. This can be done in order to obtain a desired position of a first landmark, or a first set of landmarks, relative to a position of a second landmark, or a second set of landmarks. Furthermore, segmentation of the portion of the first bone and the portion of the second bone may be done before selecting at least one of a first implant component from among a plurality of implant components. This facilitates selecting an implant component that is most optimal to achieve the desired outcome of the surgery as defined by moving the portions of bone to desired relative positions.

In some embodiments, such as in the embodiments described above, the information obtained from the first landmark and said second landmark may be obtained after segmenting the portion of the first bone and the portion of the second bone and after the portion of the first bone is moved relative to the portion of the second bone. For example, the information obtained from the first landmark and the second landmark may comprise obtaining at least one dimension, which includes at least one of diameter, length, width, and angle. As one exemplary embodiment and as illustrated in Figs. 7-8, a length may be a desired leg length, a desired offset distance (length), a length of the femur canal, a width of the femur canal, the diameter of the acetabulum, an angle of the femur canal relative a transverse plane etc. The leg length can be determined as discussed above by placing the left and right tibia landmarks 115, 116 at the same transverse plane. Alternatively or additionally, the length of the right leg (healthy side to the left in Figs. 7-8) can be determined using landmarks at the healthy side. The length of the left leg (side subject to planned treatment to the right in Figs. 7-8) can be determined using the corresponding landmarks of the treatment side. If there is a discrepancy, the bone portions of the side to be treated can be moved to achieve the desired leg length. In Fig. 7, the leg to be treated has been extended, which can be seen as the silhouette 201. Furthermore, the length and/or angle from the landmark at the proximal femur shaft 103, 104 to the landmark at the femur head 101, 102 may be determined. Also, the diameter of the femur shaft at the landmark of the proximal femur shaft 103, 104, and/or the distal femur shaft 105, 105 can be determined. These are all values that can be used in order to select the most optimal femur component 201, 202 that fits the relative positions to which the portion of first bone and the portion of second bone have been moved, as is illustrated in Fig. 7. Also, the landmark of the femur head can be used to select an appropriate cup/shell 201. For example, the diameter of the femur head can be used to select an appropriate cup/shell 201 to fit the desired anatomy. Hence, when dimensions are determined after the portions of bone have been segmented and after the bone portions have been moved into desired relative positions, selection of optimal implant components 201, 202 and relative positons are facilitated.

As is elucidated with the embodiments described above, at least one implant component 201, 202 can be selected from among a plurality of implant components in a database based on information obtained from a first landmark and a second landmark 100-121. Also, further implant components 201, 202, such as a second implant component, can be selected based on the information obtained from the first landmark and the second landmark. The landmarks used for said selecting can be landmarks of the portion of first bone or the portion of second bone. Additionally or alternatively, the first landmark is a landmark of the portion of first bone and the second landmark is a landmark of the portion of second bone. Furthermore, a third landmark of at least one of the portion of the portion of the first bone and the portion of the second bone and be identified, and information based of said first landmark, said second landmark, and said third landmark can be obtained, as has been described in the embodiments illustrated in Figs. 7-8.

In some embodiments, each implant component that is available for planning can be stored in the database 16 together with implant information. Implant information may comprise at least one of size, diameter, length, width, angle, type of material, surface treatment, etc.

According to embodiments, such as in the embodiments described above, selecting at least one of the first implant component and the second implant component may comprises selecting an implant component having implant information with a best fit to the information obtained from the first landmark and said second landmark. Thus, diameter, length, width etc. can be obtained from the landmarks 100-121, and these dimensions can be used to retrieve an implant component that has dimensions that most closely matches the dimensions determined based on the landmarks. This means that the person performing the planning does not have to try which implant component fits best, rather can focus of obtaining the desired outcome of the surgery by moving portions of bone relative to each other. Optimal components are then determined based on dimensions obtained from the landmarks after the portions of bone have been moved. The user input may be acceptance of a desired location of bone portions after surgery. This may determine positons of landmarks. These locations of landmarks can be used to determine dimensions, which in turn can be used to select optimal implant components.

Some embodiments comprises a computer readable storage medium 12, which has program instructions stored therein, wherein the program instructions, when executed by the processor 11, perform the method as described above.

As will be apparent, the features and attributes of the specific embodiments disclosed above may be combined in different ways to form additional embodiments, all of which fall within the scope of the present disclosure.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

Any process descriptions, elements, or blocks in the flow diagrams described herein and/or depicted in the attached figures should be understood as potentially representing modules, segments, or portions of code which include one or more executable instructions for implementing specific logical functions or steps in the process. Alternate implementations are included within the scope of the embodiments described herein in which elements or functions may be deleted, executed out of order from that shown or discussed, including substantially concurrently or in reverse order, depending on the functionality involved, as would be understood by those skilled in the art.

It should be emphasized that many variations and modifications may be made to the above-described embodiments, the elements of which are to be understood as being among other acceptable examples. All such modifications and variations are intended to be included herein within the scope of this disclosure and protected by the following claims.

The present invention has been described above with reference to specific embodiments. However, other embodiments than the above described are equally possible within the scope of the invention. Different method steps than those described above may be provided within the scope of the invention. The different features and steps of the invention may be combined in other combinations than those described. The scope of the invention is only limited by the appended patent claims.

## Claims

1. A computer implemented method for planning an orthopedic procedure, comprising:
retrieving (1) medical imaging data comprising a portion of a first bone and a portion of a second bone, wherein the portion of the first bone and the portion of the second bone are unsegmented;
identifying (2) within the medical imaging data a plurality of anatomical landmarks (100-121) including at least a first anatomical landmark at the portion of the first bone and at least a second anatomical landmark at the portion of the second bone;
segmenting (3) the portion of the first bone and the portion of the second bone, such that said portion of the first bone is moveable relative said portion of the second bone;
moving said portion of the first bone relative to said portion of the second bone to obtain a desired position of said first anatomical landmark relative to a position of said second anatomical landmark;
selecting (4) at least one of a first implant component (201) and a second implant component (202) from among a plurality of implant components in a database (16), wherein said selection is based on information obtained from said first anatomical landmark and said second anatomical landmark after said portion of the first bone is moved relative to said portion of the second bone; and
fitting (5) at least one of said first implant component (201) and said second implant component (202) in a space at least partially defined by said first anatomical landmark and said second anatomical landmark.

2. The method of claim 1, wherein said moving is based on at least one anatomical landmark of said plurality of anatomical landmarks (100-121), or based on a contour of said segmented portion of the first bone and/or a contour of the segmented portion of the second bone.

3. The method of any of the previous claims, further comprising identifying a third anatomical landmark of at least one of the portion of the first bone and the portion of the second bone, and obtaining said information based on said first anatomical landmark, said second anatomical landmark, and said third anatomical landmark.

4. The method of any of the previous claims, wherein obtaining said information from said first anatomical landmark and said second anatomical landmark comprises obtaining at least one dimension, which includes at least one of diameter, length, width, and angle.

5. The method of any of the previous claims, comprising storing each implant component in said database together with implant information, which optionally may include at least one of diameter, length, width, and angle.

6. The method of claim 5, wherein selecting at least one of said first implant component (201) and said second implant component (202) comprises selecting an implant component having implant information with a best fit to said information obtained from said first anatomical landmark and said second anatomical landmark.

7. A computer readable storage medium, which has program instructions stored therein, wherein the program instructions, when executed by a processor, perform the method of any one of claims 1-6.

8. An apparatus (10) for planning an orthopedic procedure, wherein the apparatus is configured to:
access a memory (12) to retrieve medical imaging data comprising a portion of a first bone and a portion of a second bone, wherein the portion of the first bone and the portion of the second bone are unsegmented;
using a processing unit (11) to identify a plurality of landmarks (100-121) including at least a first landmark at the portion of the first bone and at least a second landmark at the portion of the second bone;
using the processing unit (11) to segment the portion of the first bone and the portion of the second bone, such that said the portion of the first bone is moveable relative said portion of the second bone portion;
using the processing unit (11) to move said portion of the first bone relative to said portion of the second bone to obtain a desired position of said first anatomical landmark relative to a position of said second anatomical landmark;
using the processing unit (11) to select at least one of a first implant component (201) and a second implant component (202) from among a plurality of implant components in a database based on information obtained from said first landmark and said second landmark after said portion of the first bone is moved relative to said portion of the second bone; and
using the processing unit (11) to fit at least one of the first implant component (201) and the second implant component (202) in a space at least partially defined by said first landmark and said second landmark.

9. The apparatus of claim 8, wherein the processing unit (11) is configured to perform said move based on at least one landmark of said plurality of landmarks (100-121), or based on a contour of said segmented portion of the first bone and/or a contour of the portion of the second bone.

## Patentansprüche

1. Computerimplementiertes Verfahren zur Planung eines orthopädischen Eingriffs, aufweisend:
Abrufen (1) medizinischer Bildgebungsdaten, die einen Abschnitt eines ersten Knochens und einen Abschnitt eines zweiten Knochens aufweisen, wobei der Abschnitt des ersten Knochens und der Abschnitt des zweiten Knochens unsegmentiert sind;
Identifizieren (2) einer Vielzahl anatomischer Landmarken (100-121) innerhalb der medizinischen Bildgebungsdaten, wobei die Vielzahl anatomischer Landmarken zumindest eine erste anatomische Landmarke an dem Abschnitt des ersten Knochens und zumindest eine zweite anatomische Landmarke an dem Abschnitt des zweiten Knochens aufweist;
Segmentieren (3) des Abschnitts des ersten Knochens und des Abschnitts des zweiten Knochens, sodass der Abschnitt des ersten Knochens relativ zum Abschnitt des zweiten Knochens bewegbar ist;
Bewegen des Abschnitts des ersten Knochens relativ zum Abschnitt des zweiten Knochens, um eine gewünschte Position der ersten anatomischen Landmarke relativ zu einer Position der zweiten anatomischen Landmarke zu erhalten;
Auswählen (4) zumindest einer ersten Implantatkomponente (201) und einer zweiten Implantatkomponente (202) aus einer Vielzahl von Implantatkomponenten in einer Datenbank (16), wobei die Auswahl auf Informationen basiert, die aus der ersten anatomischen Landmarke und der zweiten anatomischen Landmarke gewonnen werden, nachdem der Abschnitt des ersten Knochens relativ zum Abschnitt des zweiten Knochens bewegt wurde; und
Anpassen (5) zumindest einer von der ersten Implantatkomponente (201) und der zweiten Implantatkomponente (202) in einen Raum, der zumindest teilweise durch die erste anatomische Landmarke und die zweite anatomische Landmarke definiert ist.

2. Verfahren nach Anspruch 1, wobei das Bewegen auf zumindest einer anatomischen Landmarke der Vielzahl anatomischer Landmarken (100-121) basiert oder auf einer Kontur des segmentierten Abschnitts des ersten Knochens und/oder einer Kontur des segmentierten Abschnitts des zweiten Knochens basiert.

3. Verfahren nach einem der vorhergehenden Ansprüche, ferner aufweisend das Identifizieren einer dritten anatomischen Landmarke von zumindest einem des Abschnitts des ersten Knochens und des Abschnitts des zweiten Knochens, sowie das Gewinnen der Informationen auf Grundlage der ersten anatomischen Landmarke, der zweiten anatomischen Landmarke und der dritten anatomischen Landmarke.

4. Verfahren nach einem der vorhergehenden Ansprüche, wobei das Gewinnen der Informationen aus der ersten anatomischen Landmarke und der zweiten anatomischen Landmarke das Gewinnen von zumindest einer Abmessung aufweist, die zumindest einen von Durchmesser, Länge, Breite und Winkel mit einschließt.

5. Verfahren nach einem der vorhergehenden Ansprüche, aufweisend das Speichern jeder Implantatkomponente in der Datenbank zusammen mit Implantatinformationen, die optional zumindest einen von Durchmesser, Länge, Breite und Winkel mit einschließen.

6. Verfahren nach Anspruch 5, wobei das Auswählen zumindest einer von der ersten Implantatkomponente (201) und der zweiten Implantatkomponente (202) das Auswählen einer Implantatkomponente aufweist, deren Implantatinformationen die beste Übereinstimmung mit den Informationen aufweisen, die aus der ersten anatomischen Landmarke und der zweiten anatomischen Landmarke gewonnen wurden.

7. Computerlesbares Speichermedium, das darin gespeicherte Programmanweisungen aufweist, wobei die Programmanweisungen, wenn sie von einem Prozessor ausgeführt werden, das Verfahren nach einem der Ansprüche 1-6 ausführen.

8. Vorrichtung (10) zur Planung eines orthopädischen Eingriffs, wobei die Vorrichtung dazu eingerichtet ist:
auf einen Speicher (12) zuzugreifen, um medizinische Bildgebungsdaten abzurufen, die einen Abschnitt eines ersten Knochens und einen Abschnitt eines zweiten Knochens aufweisen, wobei der Abschnitt des ersten Knochens und der Abschnitt des zweiten Knochens unsegmentiert sind;
unter Verwendung einer Verarbeitungseinheit (11) eine Vielzahl von Landmarken (100-121) zu identifizieren, wobei die Vielzahl von Landmarken zumindest eine erste Landmarke an dem Abschnitt des ersten Knochens und zumindest eine zweite Landmarke an dem Abschnitt des zweiten Knochens aufweist;
unter Verwendung der Verarbeitungseinheit (11) den Abschnitt des ersten Knochens und den Abschnitt des zweiten Knochens zu segmentieren, sodass der Abschnitt des ersten Knochens relativ zu dem Abschnitt des zweiten Knochens bewegbar ist;
unter Verwendung der Verarbeitungseinheit (11) den Abschnitt des ersten Knochens relativ zu dem Abschnitt des zweiten Knochens zu bewegen, um eine gewünschte Position der ersten anatomischen Landmarke relativ zu einer Position der zweiten anatomischen Landmarke zu erhalten;
unter Verwendung der Verarbeitungseinheit (11) zumindest eine von einer ersten Implantatkomponente (201) und einer zweiten Implantatkomponente (202) aus einer Vielzahl von Implantatkomponenten in einer Datenbank auszuwählen, basierend auf Informationen, die aus der ersten Landmarke und der zweiten Landmarke gewonnen werden, nachdem der Abschnitt des ersten Knochens relativ zu dem Abschnitt des zweiten Knochens bewegt wurde; und
unter Verwendung der Verarbeitungseinheit (11) zumindest eine von der ersten Implantatkomponente (201) und der zweiten Implantatkomponente (202) in einen Raum einzupassen, der zumindest teilweise durch die erste Landmarke und die zweite Landmarke definiert ist.

9. Vorrichtung nach Anspruch 8, wobei die Verarbeitungseinheit (11) dazu eingerichtet ist, die Bewegung auf Grundlage zumindest einer Landmarke der Vielzahl von Landmarken (100-121) oder auf Grundlage einer Kontur des segmentierten Abschnitts des ersten Knochens und/oder einer Kontur des Abschnitts des zweiten Knochens durchzuführen.

## Revendications

1. Procédé mis en œuvre par ordinateur pour planifier une intervention orthopédique, comprenant :
l'extraction (1) de données d'imagerie médicale comprenant une partie d'un premier os et une partie d'un second os, dans lequel la partie du premier os et la partie du second os sont non segmentées ;
l'identification (2), au sein des données d'imagerie médicale, d'une pluralité de repères anatomiques (100-121) incluant au moins un premier repère anatomique au niveau de la partie du premier os et au moins un deuxième repère anatomique au niveau de la partie du second os ;
la segmentation (3) de la partie du premier os et de la partie du second os, de sorte que ladite partie du premier os soit mobile par rapport à ladite partie du second os ;
le déplacement de ladite partie du premier os par rapport à ladite partie du second os pour obtenir une position souhaitée dudit premier repère anatomique par rapport à une position dudit deuxième repère anatomique ;
la sélection (4) d'au moins l'un d'un premier composant d'implant (201) et d'un second composant d'implant (202) parmi une pluralité de composants d'implant dans une base de données (16), dans lequel ladite sélection repose sur des informations obtenues à partir dudit premier repère anatomique et dudit deuxième repère anatomique après déplacement de ladite partie du premier os par rapport à ladite partie du second os ; et
l'insertion (5) d'au moins l'un dudit premier composant d'implant (201) et dudit second composant d'implant (202) dans un espace au moins partiellement défini par ledit premier repère anatomique et ledit deuxième repère anatomique.

2. Procédé selon la revendication 1, dans lequel ledit déplacement est basé sur au moins un repère anatomique de ladite pluralité de repères anatomiques (100-121), ou est basé sur un contour de ladite partie segmentée du premier os et/ou un contour de la partie segmentée du second os.

3. Procédé selon l'une quelconque des revendications précédentes, comprenant en outre l'identification d'un troisième repère anatomique d'au moins l'une de la partie du premier os et de la partie du second os, et l'obtention desdites informations sur la base dudit premier repère anatomique, dudit deuxième repère anatomique et dudit troisième repère anatomique.

4. Procédé selon l'une quelconque des revendications précédentes, dans lequel l'obtention desdites informations à partir dudit premier repère anatomique et dudit deuxième repère anatomique comprend l'obtention d'au moins une dimension, qui inclut au moins un élément parmi un diamètre, une longueur, une largeur et un angle.

5. Procédé selon l'une quelconque des revendications précédentes, comprenant le stockage de chaque composant d'implant dans ladite base de données conjointement avec des informations d'implant, qui peuvent éventuellement inclure au moins un élément parmi un diamètre, une longueur, une largeur et un angle.

6. Procédé selon la revendication 5, dans lequel la sélection d'au moins l'un dudit premier composant d'implant (201) et dudit second composant d'implant (202) comprend la sélection d'un composant d'implant présentant des informations d'implant les plus adaptées auxdites informations obtenues à partir dudit premier repère anatomique et dudit deuxième repère anatomique.

7. Support de stockage lisible par ordinateur, qui présente des instructions de programme stockées dans celui-ci, dans lequel les instructions de programme, lorsqu'elles sont exécutées par un processeur, exécutent le procédé selon l'une quelconque des revendications 1-6.

8. Appareil (10) de planification d'une intervention orthopédique, dans lequel l'appareil est configuré pour :
accéder à une mémoire (12) pour en extraire des données d'imagerie médicale comprenant une partie d'un premier os et une partie d'un second os, dans lequel la partie du premier os et la partie du second os sont non segmentées ;
l'utilisation d'une unité de traitement (11) pour identifier une pluralité de repères (100-121) incluant au moins un premier repère au niveau de la partie du premier os et au moins un deuxième repère au niveau de la partie du second os ;
l'utilisation de l'unité de traitement (11) pour segmenter la partie du premier os et la partie du second os, de sorte que ladite partie du premier os soit mobile par rapport à ladite partie de la seconde partie osseuse ;
l'utilisation de l'unité de traitement (11) pour déplacer ladite partie du premier os par rapport à ladite partie du second os pour obtenir une position souhaitée dudit premier repère anatomique par rapport à une position dudit deuxième repère anatomique ;
l'utilisation de l'unité de traitement (11) pour sélectionner au moins l'un d'un premier composant d'implant (201) et d'un second composant d'implant (202) parmi une pluralité de composants d'implant dans une base de données sur la base d'informations obtenues à partir dudit premier repère et dudit deuxième repère après déplacement de ladite partie du premier os par rapport à ladite partie du second os ; et
l'utilisation de l'unité de traitement (11) pour insérer au moins l'un du premier composant d'implant (201) et du second composant d'implant (202) dans un espace au moins partiellement défini par ledit premier repère et ledit deuxième repère.

9. Appareil selon la revendication 8, dans lequel l'unité de traitement (11) est configurée pour effectuer ledit déplacement sur la base d'au moins un repère de ladite pluralité de repères (100-121), ou sur la base d'un contour de ladite partie segmentée du premier os et/ou d'un contour de la partie du second os.
